(19) Europäisches Patentamt
European Patent Office
Office européen des brevets

(11) EP 0 318 029 B1

(12) **EUROPEAN PATENT SPECIFICATION**

(45) Date of publication and mention of the grant of the patent: **07.02.1996 Bulletin 1996/06**

(21) Application number: **88119675.2**

(22) Date of filing: **25.11.1988**

(51) Int. Cl.$^6$: **C07D 211/14**, C07D 211/32, C07D 211/40, C07D 401/12, C07D 211/18, C07D 211/58, C07D 223/04, A61K 31/445, A61K 31/55

# (54) Cyclic amine and pharmacological composition

Cyclische Amine und pharmakologische Verbindungen

Amines cycliques et compositions pharmaceutiques les renfermant

(84) Designated Contracting States:
**AT BE CH DE FR GB IT LI LU NL SE**

(30) Priority: **27.11.1987 JP 299438/87**
**28.03.1988 JP 74127/88**

(43) Date of publication of application:
**31.05.1989 Bulletin 1989/22**

(73) Proprietor: **Eisai Co., Ltd.**
**Tokyo (JP)**

(72) Inventors:
- **Sugimoto, Hachiro**
**Ushiku-shi Ibaraki (JP)**
- **Nakamura, Takaharu**
**Abiko-shi Chiba (JP)**
- **Karibe, Norio**
**Tsukuba-shi Ibaraki (JP)**
- **Saito, Isao**
**Tsukuba-shi Ibaraki (JP)**
- **Higurashi, Kunizo**
**Sumida-ku Tokyo (JP)**
- **Yonaga, Masahiro**
**Tsukuba-shi Ibaraki (JP)**
- **Kaneko, Takeru**
**Tsukuba-shi Ibaraki (JP)**
- **Nakazawa, Takahiro**
**Kitasouma-gun Ibaraki (JP)**
- **Ueno, Masataka**
**Tsukuba-shi Ibaraki (JP)**
- **Yamatsu, Kiyomi**
**Kamakura-shi Kanagawa (JP)**
- **Ueno, Kohshi**
**Tsukuba-shi Ibaraki (JP)**
- **Ikeda, Masuhiro**
**Tsukuba-shi Ibaraki (JP)**

(74) Representative: **Hansen, Bernd, Dr.rer.nat. et al**
**D-81904 München (DE)**

(56) References cited:
**FR-A- 2 227 868** **US-A- 3 576 810**
**US-A- 4 075 346**

- **CHEMICAL ABSTRACTS, vol. 106, no. 25, 22nd June 1987, page 645, abstract no. 213767z, Columbus, Ohio, US; & JP-A-61 227 565**
- **ARZNEIMITTELFORSCHUNG, vol. 24, no. 4a, 1974, pages 584-600; R. HILTMANN et al. : "2-Acylaminopyridin-Derivate mit morphinagonistischer und -antagonistischer Wirksamkeit"**
- **JOURNAL ORGANIC CHEMISTRY, vol. 33, no. 9, September 1968, pages 3627-3632; R. A. JOHNSON: "Conformations of alkylpiperidine amides"**
- **JOURNAL OF ORGANIC CHEMISTRY, vol. 31, no. 10, October 1966, pages 3337-3342; D.M. LYNCH et al.: "Pyridylmethylnaphthalene and pyridylmethylindan derivatives"**
- **JOURNAL OF MEDICINAL CHEMISTRY, vol. 13, no. 1, January 1970, pages 1-6, American Chemical Society; R.L. DUNCAN Jr. et al.: "Aroylpiperidines and pyrrolidines. A new class of potent central nervous system depressants"**
- **ARZNEIMITTELFORSCHUNG, vol. 39, no. 4, 1989, pages 445-450; T. KANEKO et al.: "Anti-ischemic effect of the novel anti-ischemic agent 2-(4-(p-fluorobenzoyl)- piperidin-1-yl)-2'-acetonaphthone hydrochloride and possible mechanism of its action"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.9.9/3.4

- THE JAPANESE-UNITED STATES CONGRESS OF PHARMACEUTICAL SCIENCES, POSTER, abstracts, Honolulu, 2nd-7th December 1987, page 159; N. KARIBE et al.: "Anti-ischemic effect of the novel anti-ischemic agent 2-(4(p-fluoroblenzoyl)- piperidin-1-yl)-2'-acetonaphthone hydrochloride and possible mechanism of its action"

## Description

The present invention relates to a novel cyclic amine derivative which exhibits an excellent activity as a pharmaceutical.

[Background of the Invention and Prior Art]

Various attempts have been made to treat cerebrovascular diseases with a drug. For example, cerebrovasodilators and cerebral metabolism activators are used. So far, however, there has been no drug which is very useful for the treatment of the cerebrovascular diseases. In particular, with respect to cerebrovascular dementia, mental dysfunction, etc. among various symptoms accompanying cerebrovascular diseases, no effective drug has yet been developed.

However, in recent years, a theory that an excitatory amino acid liberated after ischemia seriously participates in the death of nerve cells has attracted worldwide attention. This theory was substantiated by the facts that ischemia brings about liberation of a glutamate and the cell injury derived from ischemia is antagonized by a glutamate antagonist.

US-A-4075346 discloses derivatives useful as central nervous system depressants which include a propyl linking group between a benzoyl group and piperidene group.

The Japanese - United States Congress of Pharmaceutical Sciences, post abstracts, 2nd-7th December 1987, page 159 discloses certain piperidene derivatives which have a potent inhibitory effect on glutamate release.

EP-A-0288563, published on 7th April 1988, discloses the use of various piperidene derivatives useful for the treatment of cerebral disorder.

The present inventors have paid their attention to the neurotoxin activity of the above-described glutamate in starting the search for a new agent for treating cerebrovascular diseases, particularly an agent for improving a mental symptom accompanying the cerebrovascular diseases and have repeated studies on various compounds for a long period of time. As a result, we have found that a cyclic amine derivative which will be described hereinbelow exhibits an excellent activity on the suppression of glutamate liberation, which have led to the completion of the present invention.

Accordingly, an object of the present invention is to provide a cyclic amine derivative or pharmacologically acceptable salts thereof effective as an agent for improving mental symptoms accompanying cerebrovascular diseases, such as cerebral apoplexy, cerebral hemorrhage, cerebral infarction and cerebral arteriosclerosis, and polyinfarctive dementia, and a process for preparing said compound and pharmacologically acceptable salts thereof. Another object of the present invention is to provide a pharmaceutical comprising as an effective ingredient said compound or pharmacologically acceptable salts thereof.

According to one aspect, the present invention provides a cyclic amine or a pharmacologically acceptable salt thereof represented by the general formula:

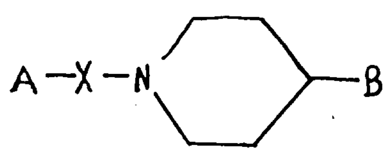

wherein A is a naphthyl group or a naphthyl group substituted by an alkoxy group having 1-6 carbon atoms or hydroxy; phenyl or a phenyl group substituted by an alkyl group having 1-6 carbon atoms or a halogen; a quinolyl group; or an alkyl group having 1-6 carbon atoms,

X is a group represented by the formula

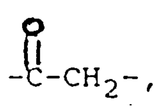

a group represented by the formula

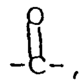

a group represented by the formula

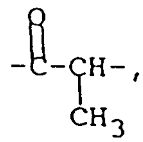

or a group represented by the formula

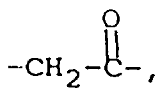

and

B is an alkyl group having 1-6 carbon atoms; or a group represented by the formula -Y-Z wherein Y represents -O- or -NH-; and

Z is a phenyl group which may be substituted by one or two of the same or different substituents selected from the group consisting of a halogen, an alkyl group having 1-6 carbon atoms, and an alkoxy group having 1-6 carbon atoms; a naphthyl group; a pyridyl group; or an alkyl group having 1-6 carbon atoms;
with the proviso that:

(i) if X represents -CO-, n=2 and B represents a methyl group, then A is neither a methyl group nor an unsubstituted phenyl group; and
(ii) if A is a methyl group, X represents $-COCH_2-$and n=2, then B is not a methyl group or a phenyl group.

According to a second aspect, the present invention provides the use of a pharmacologically effective amount of a cyclic amine or a pharmacologically acceptable salt thereof represented by the general formula:

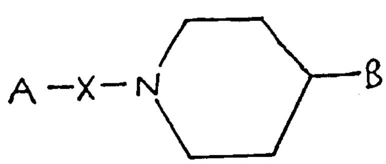

wherein A is a naphthyl group or a naphthyl group substituted by an alkoxy group having 1-6 carbon atoms or hydroxy; phenyl or a phenyl group substituted by an alkyl group having 1-6 carbon atoms or a halogen; a quinolyl group; or an alkyl group having 1-6 carbon atoms,

X is a group represented by the formula

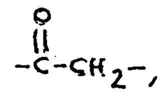

a group represented by the formula

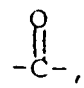

a group represented by the formula

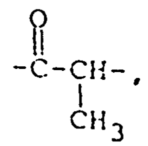

or a group represented by the formula

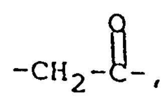

and

B is an alkyl group having 1-6 carbon atoms; or a group represented by the formula -Y-Z wherein Y represents -O- or -NH-; and

Z is a phenyl group which may be substituted by one or two of the same or different substituents selected from the group consisting of a halogen, an alkyl group having 1-6 carbon atoms, and an alkoxy group having 1-6 carbon atoms; a naphthyl group; a pyridyl group; or an alkyl group having 1-6 carbon atoms;

for the manufacture of a medicament for improving, treating or preventing a mental symptom associated with a cerebrovascular disease.

The term "alkyl group having 1-6 carbon atoms" used in the above definition of A, B, and Z with respect to the compound (I) of the presented invention is intended to mean a straight-chain or branched alkyl group having 1 to 6 carbon atoms, and examples thereof include methyl, ethyl, propyl, isopropyl, butyl, isobutyl, sec-butyl, tert- butyl, pentyl (amyl), isopentyl, neopentyl, tert-pentyl, 1-methylbutyl, 2-methylbutyl, 1,2-dimethylpropyl, hexyl, isohexyl, 1-methylbantyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethyl butyl, 1,1,2-trimethylpropyl, 1,2,2-trimethylpropyl, 1-ethyl-1-methylpropyl, and 1-ethyl-2-methylpropyl groups. Among them, methyl, ethyl, propyl, isopropyl groups etc. are preferable. A methyl group is most preferable.

The term "alkoxy group having 1-6 carbon atoms" used in the definition of A, and Z is intended to mean an alkoxy group derived from the above-described alkyl group, and preferable examples thereof include methoxy, ethoxy, propoxy, isopropoxy, butoxy, and isobutoxy groups. Among them, a methoxy group is most preferable.

Examples of the halogen atom involved in the definition of A, and Z include chlorine, bromine, fluorine, and iodine.

The term "pharmacologically acceptable salt" is intended to mean a commonly used nontoxic salt, and examples thereof include those of inorganic acids, such as hydrochloride, hydrobromide, sulfate and phosphate, those of organic acids, such as acetate, maleate, tartrate, methanesulfonate, benzenesulfonate, and toluenesulfonate, and salts with amino acids such as arginine and aspartic acid.

In preferable embodiments of the cyclic amine and pharmacologically acceptable salts thereof as defined above, (1) B is a lower alkyl; (2) B is -Y-Z in which Y is -O-; or (3) A is a naphthyl group or a naphthyl group having a substituent

of a lower alkoxy or hydroxy, X is -CO-CH2-, and B is -Y-Z in which Y is -O-or -NH- and Z is phenyl or phenyl having one or two substituents, either the same as or different from each other, selected from the group consisting of a halogen, a lower alkyl, a lower alkoxyl and naphthyl.

The invention provides a process for preparing a cyclic amine or a pharmacologically acceptable salt thereof as defined above, which comprises the step of reacting a halide compound having the formula: A-X-Hal in which Hal is a halogen and A and X each are defined above, with a compound having the formula:

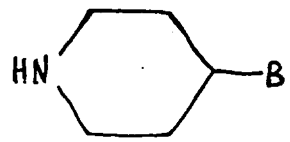

( III )

in which B is as defined above.

The invention also provides a pharmacological composition which comprises a pharmacologically effective amount of a cyclic amine or a pharmacologically acceptable salt thereof as defined above and a pharmacologically acceptable carrier.

The compounds of the present invention may be prepared by various processes. A representative process for preparing the compounds of the present invention will now be described.

<u>Process of preparation</u>

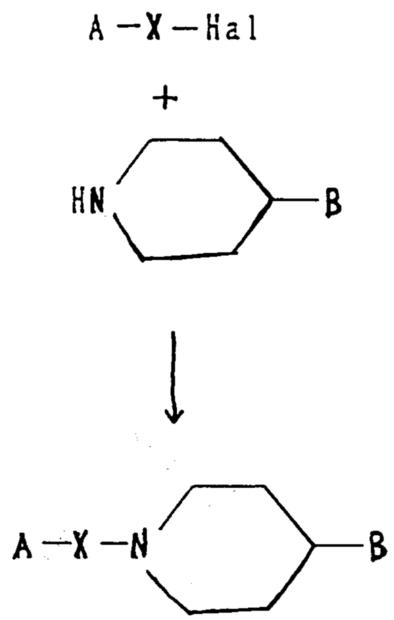

( II )

( III )

( I )

wherein A, X and B are as defined above and Hal is a halogen atom.

Specifically, a halide represented by the general formula (II) is reacted with a compound represented by the general formula (III) to prepare a compound (I) which is an object compound of the present invention.

This reaction is dehydrohalogenation conducted by any ordinary method while heating in the absence of any solvent or in an organic solvent which does not participate in the reaction, i.e., one selected from among alcoholic solvents, such as methanol, ethanol, and butanol, benzene, toluene, xylene, tetrahydrofuran, chloroform, carbon tetrachloride, and dimethylformamide. In this case, favorable results can be attained by conducting the reaction in the present of an inorganic salt, such as sodium hydrogencarbonate, potassium carbonate, sodium carbonate or caustic soda, or an organic base such as triethylamine, pyridine, pyrimidine or diethylaniline.

The effect of the present invention will now be described in more detail with reference to the following pharmacological experiment.

Experimental Example

Glutamate liberation inhibitory activity in cerebral cortex slice

A male SD rat was decapitated, and cerebral cortices on both sides were then extirpated to prepare a slice having a thickness of 750 µm (weight: 10 to 20 mg) with a McIlwain tissue chopper. The slice was incubated in a Krebs-Henseleit liquid containing a test compound in the form of a solution thereof for 30 minutes. Then the slice was incubated in one ml of a Krebs-Henseleit liquid containing 50 mM of KCl and the test compound solution for 60 mins. For control, a solvent was added to a Krebs-Henseleit liquid containing only 50 mM of KCl.

The glutamate liberated in the solution was determined by HPLC, and the glutamate liberation inhibitory activity (%) in $10^{-4}$ M of each test compound solution was calculated by the following equation:

$$\left(1 - \frac{\text{glutamate in each test compound solution}}{\text{amount of glutamate in control group}}\right) \times 100$$

Table 1

| glutamate liberation inhibitory activity | |
|---|---|
| test compound | glutamate liberation inhibitory activity (%) 1 x $10^{-4}$ M |
| compd. of Ex. 3 | 49 |

It is apparent from the above-described pharmacological experiment that the compounds of the present invention have a remarkable glutamate liberation inhibitory activity and exhibit a cell injury protective action and a learning disturbance improving action after ischemia based on this activity. Therefore, the compounds of the present invention exhibit a useful pharmacological action, particularly a remarkable ischemic cerebrovascular disease improving action for the central nervous system and are useful as an agent for improving, treating or preventing mental symptoms associated with cerebrovascular diseases such as cerebral apoplexy, cerebral hemorrhage, cerebral infarction, cerebral arteriosclerosis, and various types of dementia such as polyinfarcted dementia.

Further, the compounds of the present invention exhibited high safety in toxicity tests on rats. Therefore, the present invention is valuable from this viewpoint as well.

When the compounds of the present invention are used as a pharmaceutical for the above-described diseases, they may be orally or parenterally administered. The dose will vary depending on the severity of symptome, age, sex, weight and sensitivity of patients; method of administration; time and intervals of administration and properties, formulation, and kind of pharmaceutical preparations; kind of effective ingredients, etc., so that there is no particular limitation with respect to the dose. Normally, the compounds may be administered in a dose of about 0.1 to 300 mg, preferably about 1 to 100 mg, per day per adult, ordinarily in one to four portions.

The compounds of the present invention are converted into pharmaceutical preparations in the dosage form of, e.g., injections, suppositories, sublingual tablets, and capsules according to any method which is commonly accepted in the art.

In preparing injections, the effective ingredient is blended, if necessary, with a pH modifier, a buffer, a suspending agent, a solubilizing agent, a stabilizer, a tonicity agent, a preservative, etc., followed by preparation of an intravenous, subcutaneous, or intramuscular injection according to an ordinary method. In this case, if necessary, it is possible to lyophilize these preparations according to any ordinary method.

Examples of the suspending agent include methylcellulose, Polysorbate 80, hydroxyethylcellulose, gum arabic, powdered trangacanth, sodium carboxymethylcellulose, and polyoxyethylene sorbitan monolaurate.

Examples of the solubilizing agent include polyoxyethylene hydrogenated castor oil, Polysorbate 80, nicotinamide, polyoxyethylene sorbitan monolaurate, Macrogol, and ethyl esters of castor oil fatty acids.

Examples of the stabilizer include sodium sulfite, sodium metasulfite, and ether, and examples of the preservative include methyl p-hydroxybenzoate, ethyl p-hydroxybenzoate, sorbic acid, phenol, cresol, and chlorocresol.

[Examples]

Representative examples of the present invention will now be described for the purpose of aiding the understanding of the present invention.

Example 1

2-(4-Methylpiperidinyl)-2'-acetonaphthone hydrochloride

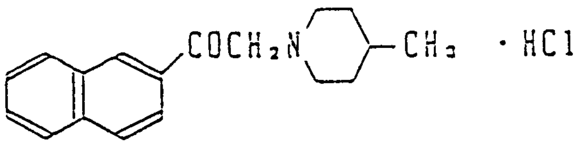

2.5 g of 2-bromo-2'-acetophthone, 1 g of methylpiperidine, 0.1 g of potassium iodide, and 3.1 g of triethylamine were added to 100 ml of ethanol. The mixture was refluxed for 20 min. The solvent was distilled off, and dichloromethane was added to the residue. The mixture was washed with water and dried. The dichloromethane was distilled off, and the residue was purified by column chromatography (silica gel) to prepare an oleaginous and oily intended compound. The compound was converted into a hydrochloride by an oridinary method and then recrystallized to prepare 1.87 g of an intended hydrochloride.

- melting point: 202 - 203.5°C
- elementary analysis: $C_{18}H_{21}NO{\cdot}HC\ell$

| | C | H | N |
|---|---|---|---|
| calculated (%): | 71.16 | 7.30 | 4.61 |
| found (%): | 71.21 | 7.59 | 4.50 |

Examples 2 to 4

Various compounds shown in Table 2 were synthesized in the same manner as that of Example 1.

Table 2

| Ex. No. | structural formula | m.p. (°C) | chemical formula | elem. anal. calcd. (%) found (%) C | H | N |
|---|---|---|---|---|---|---|
| 2 | 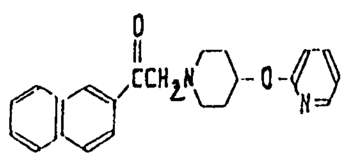 · 2HCℓ | 125~126 | $C_{23}H_{22}N_2O_2 \cdot 2HCl$ | 63.01<br>62.90 | 5.77<br>5.52 | 6.68<br>6.83 |

Table 2 (continued)

| Ex. No. | structural formula | m.p. (°C) | chemical formula | elem. anal. calcd. (%) found (%) | | |
|---|---|---|---|---|---|---|
| | | | | C | H | N |
| 3 | 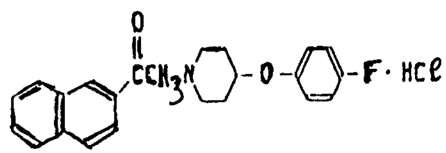 | 224.5~225 | $C_{23}H_{22}NO_2 \cdot HCl$ | 69.08<br>68.95 | 5.80<br>5.76 | 3.50<br>3.26 |

Table 2 (continued)

| Ex. No. | structural formula | m.p. (°C) | chemical formula | elem. anal. calcd. (%) found (%) | | |
|---|---|---|---|---|---|---|
| | | | | C | H | N |
| 4 | 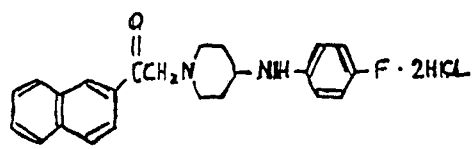 | 253.5~254 | $C_{24}H_{23}N_2OF \cdot 2HCL$ | 63.45<br>63.17 | 5.79<br>5.69 | 6.43<br>6.20 |

## Claims

1. A cyclic amine or a pharmacologically acceptable salt thereof represented by the general formula:

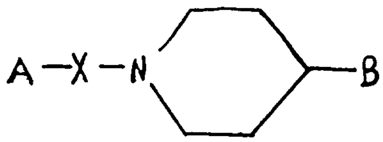

wherein A is a naphthyl group or a naphthyl group substituted by an alkoxy group having 1-6 carbon atoms of hydroxy; phenyl or a phenyl group substituted by an alkyl group having 1-6 carbon atoms or a halogen; a quinolyl group; or an alkyl group having 1-6 carbon atoms,
X is a group represented by the formula

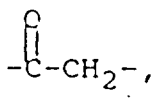

a group represented by the formula

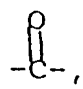

a group represented by the formula

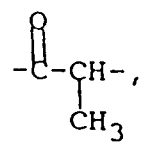

or a group represented by the formula

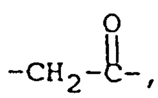

and
B is an alkyl group having 1-6 carbon atoms; or a group represented by the formula -Y-Z wherein Y represents -O- or -NH-; and
Z is a phenyl group which may be substituted by one or two of the same or different substituents selected from the group consisting of a halogen, an alkyl group having 1-6 carbon atoms, and an alkoxy group having 1-6 carbon atoms; a naphthyl group; a pyridyl group; or an alkyl group having 1-6 carbon atoms;
with the proviso that:

(i) if X represents -CO-, n=2 and B represents a methyl group, then A is neither a methyl group nor an unsubstituted phenyl group; and
(ii) if A is a methyl group, X represents $-COCH_2-$and n=2, then B is not a methyl group or a phenyl group.

2. A cyclic amine or a pharmacologically acceptable salt thereof according to claim 1, wherein B is an alkyl group having 1-6 carbon atoms.

3. A cyclic amine or a pharmacologically acceptable salt thereof according to claim 1, wherein B is -Y-Z and Y is -O-.

4. A cyclic amine or a pharmacologically acceptable salt thereof according to claim 1, wherein A is a naphthyl group or a naphthyl group having a substituent of an alkoxy group having 1-6 carbon atoms or hydroxy, X is $-CO-CH_2-$, and B is -Y-Z in which Y is -O- or -NH- and Z is phenyl or phenyl having one or two substituents which may be the same or different from each other, selected from a halogen, an alkyl group having 1-6 carbon atoms, an alkoxy group having 1-6 carbon atoms and naphthyl.

5. A process for preparing a cyclic amine or a pharmacologically acceptable salt thereof as defined in claim 1, which comprises the step of reacting a halide compound having the formula: A-X-Hal in which Hal is a halogen and A and X are each as defined in claim 1, with a compound having the formula:

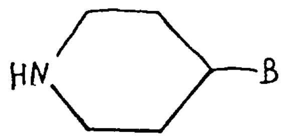

in which B is as defined in claim 1.

6. A pharmaceutical composition which comprises a pharmacologically effective amount of a cyclic amine or a pharmacologically acceptable salt thereof as defined in claim 1 and a pharmacologically acceptable carrier.

7. The use of a pharmacologically effective amount of a cyclic amine or a pharmacologically acceptable salt thereof represented by the general formula:

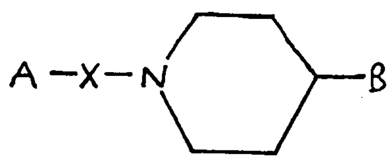

wherein A is a naphthyl group or a naphthyl group substituted by an alkoxy group having 1-6 carbon atoms or hydroxy; phenyl or a phenyl group substituted by an alkyl group having 1-6 carbon atoms or a halogen; a quinolyl group; or an alkyl group having 1-6 carbon atoms,
X is a group represented by the formula

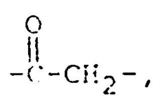

a group represented by the formula

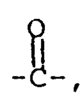

a group represented by the formula

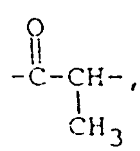

or a group represented by the formula

$$-CH_2-\overset{\displaystyle O}{\overset{\|}{C}}-,$$

and

B is an alkyl group having 1-6 carbon atoms; or a group represented by the formula -Y-Z wherein Y represents -O- or -NH-; and

Z is a phenyl group which may be substituted by one or two of the same or different substituents selected from the group consisting of a halogen, an alkyl group having 1-6 carbon atoms, and an alkoxy group having 1-6 carbon atoms; a naphthyl group; a pyridyl group; or an alkyl group having 1-6 carbon atoms;
for the manufacture of a medicament for improving, treating or preventing a mental symptom associated with a cerebrovascular disease.

**Patentansprüche**

1. Ein cyclisches Amin oder ein pharmakologisch annehmbares Salz davon, repräsentiert durch die allgemeine Formel:

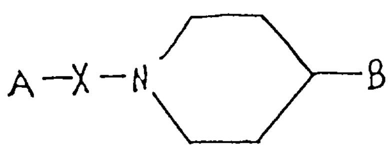

worin A ein Naphthyl-Gruppe oder eine Naphthyl-Gruppe, die mit einer Alkoxy-Gruppe mit 1 - 6 Kohlenstoffatomen oder einer Hydroxy-Gruppe substituiert ist; eine Phenyl-Gruppe oder eine Phenyl-Gruppe, die mit einer Alkyl-Gruppe mit 1 - 6 Kohlenstoffatomen oder einem Halogenatom substituiert ist; eine Chinolyl-Gruppe, oder eine Alkyl-Gruppe mit 1 bis 6 Kohlenstoffatomen darstellt; X ist eine Gruppe repräsentiert durch die Formel

$$-\overset{\displaystyle O}{\overset{\|}{C}}-CH_2-,$$

eine Gruppe repräsentiert durch die Formel

$$-\overset{\displaystyle O}{\overset{\|}{C}}-$$

eine Gruppe, repräsentiert durch die Formel

$$-\overset{\displaystyle O}{\overset{\|}{C}}-\underset{\displaystyle CH_3}{\underset{|}{CH}}-$$

oder eine Gruppe, repräsentiert durch die Formel

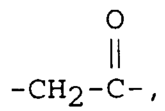

und B ist eine Alkyl-Gruppe mit 1 - 6 Kohlenstoffatomen oder eine Gruppe, repräsentiert durch die Formel -Y-Z, worin Y -O- oder -NH- repräsentiert, und Z ist eine Phenyl-Gruppe, die substituiert sein kann mit ein oder zwei identischen oder verschiedenen Substituenten ausgewählt aus der Gruppe bestehend aus einem Halogen, einer Alkyl-Gruppe mit 1 - 6 Kohlenstoffatomen, und einer Alkoxy-Gruppe mit 1 - 6 Kohlenstoffatomen; eine Naphthyl-Gruppe; eine Pyridyl-Gruppe; oder eine Alkyl-Gruppe mit 1 - 6 Kohlenstoffatomen; mit der Einschränkung, daß:

(i) falls X -CO- repräsentiert, n=2 und B eine Methyl-Gruppe repräsentiert, a weder eine Methyl-Gruppe noch eine unsubstituiert Phenyl-Gruppe ist, und

(ii) falls A eine Methyl-Gruppe ist, X $-COCH_2-$repräsentiert und n=2, B nicht eine Methyl-Gruppe oder eine Phenyl-Gruppe ist.

**2.** Ein cyclisches Amin oder ein pharmakologisch annehmbares Salz davon gemäß Anspruch 1,
dadurch **gekennzeichnet,**
daß B eine Alkyl-Gruppe mit 1 - 6 Kohlenstoffatomen ist.

**3.** Ein cyclisches Amin oder ein pharmakologisch annehmbares Salz davon gemäß Anspruch 1,
dadurch **gekennzeichnet,**
daß B -Y-Z ist und Y ist -O-.

**4.** Ein cyclisches Amin oder ein pharmakologisch annehmbares Salz davon gemäß Anspruch 1,
dadurch **gekennzeichnet,**
daß A eine Naphthyl-Gruppe ist oder eine Naphthyl-Gruppe mit einem Substituenten, der eine Alkoxy-Gruppe mit 1 - 6 Kohlenstoffatomen oder eine Hydroxy-Gruppe ist, X ist $-CO-CH_2-$, und B is -Y-Z, worin Y -O- oder -NH- ist, und Z ist Phenyl oder Phenyl mit einem oder zwei Substituenten, die gleich oder voneinander verschieden sein können, ausgewählt aus einem Halogen, einer Alkyl-Gruppe mit 1 - 6 Kohlenstoffatomen, einer Alkoxy-Gruppe mit 1 - 6 Kohlenstoffatomen und Naphthyl.

**5.** Verfahren zur Herstellung eines cyclischen Amins oder eines pharmakologisch annehmbaren Salzes davon, wie in Anspruch 1 definiert, umfassend den Schritt der Reaktion einer Halogen-Verbindung der Formel A-X-Hal, worin Hal ein Halogen ist und A und X sind wie in Anspruch 1 definiert, mit einer Verbindung der Formel:

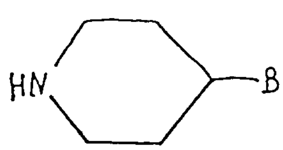

worin B wie in Anspruch 1 definiert ist.

**6.** Eine pharmazeutische Zusammensetzung, umfassend eine pharmakologisch wirksame Menge eines cyclischen Amins oder eines pharmakologisch annehmbaren Salzes davon wie in Anspruch 1 definiert und einen pharmakologisch annehmbaren Träger.

7. Verwendung einer pharmakologisch wirksamen Menge eines cyclischen Amins oder eines pharmakologisch annehmbaren Salzes davon, das repräsentiert wird durch die allgemein Formel:

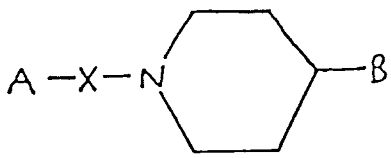

worin A ein Naphthyl-Gruppe oder eine Naphthyl-Gruppe, die mit einer Alkoxy-Gruppe mit 1 - 6 Kohlenstoffatomen oder einer Hydroxy-Gruppe substituiert ist; eine Phenyl-Gruppe oder eine Phenyl-Gruppe, die mit einer Alkyl-Gruppe mit 1 - 6 Kohlenstoffatomen oder einem Halogenatom substituiert ist; eine Chinolyl-Gruppe; oder eine Alkyl-Gruppe mit 1 bis 6 Kohlenstoffatomen darstellt, X ist eine Gruppe repräsentiert durch die Formel

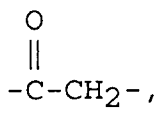

eine Gruppe repräsentiert durch die Formel

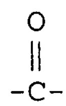

eine Gruppe, repräsentiert durch die Formel

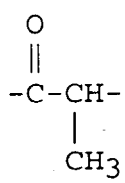

oder eine Gruppe, repräsentiert durch die Formel

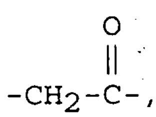

und B ist eine Alkyl-Gruppe mit 1 - 6 Kohlenstoffatomen oder eine Gruppe repräsentiert durch die Formel -Y-Z, worin Y -O- oder -NH- repräsentiert, und Z ist eine Phenyl-Gruppe, die substituiert sein kann mit ein oder zwei identischen oder verschiedenen Substituenten ausgewählt aus der Gruppe bestehend aus einem Halogen, einer Alkyl-Gruppe mit 1 - 6 Kohlenstoffatomen, und einer Alkoxy-Gruppe mit 1 - 6 Kohlenstoffatomen; eine Naphthyl-Gruppe; eine Pyridyl-Gruppe; oder eine Alkyl-Gruppe mit 1 - 6 Kohlenstoffatomen;
zur Herstellung eines Medikaments zur Verbesserung, Behandlung oder Vorbeugung von mentalen Symptomen, die mit einer cerebrovaskulären Krankheit assoziiert sind.

## Revendications

**1.** Amine cyclique ou sel pharmacologiquement acceptable dérive de celle-ci, représentée par la formule générale :

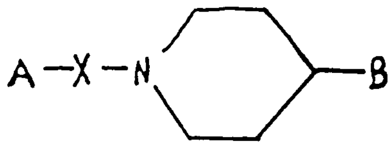

dans laquelle A représente un groupe naphtyle ou un groupe naphtyle substitué avec un groupe alkoxy comportant de 1 à 6 atomes de carbone ou un groupe hydroxy ; un groupe phényle ou un groupe phényle substitué avec un groupe alkyle comportant de 1 à 6 atomes de carbone ou un atome d'halogène ; un groupe quinolyle ; ou un groupe alkyle comportant de 1 à 6 atomes de carbone,
X représente un groupe de formule

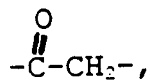

un groupe
de formule

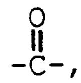

un groupe de formule

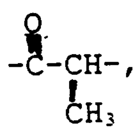

ou un groupe de formule

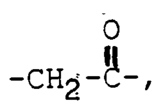

et
B représente un groupe alkyle comportant de 1 à 6 atomes de carbone ; ou un groupe de formule -Y-Z dans laquelle Y représente -O- ou -NH- ; et
Z représente un groupe phényle qui peut être substitué avec 1 ou 2 substituants identiques ou différents, choisis parmi un atome d'halogène, un groupe alkyle comportant de 1 a 6 atomes de carbone et un groupe alkoxy comportant de 1 à 6 atomes de carbone ; un groupe naphtyle ; un groupe pyridyle ; ou un groupe alkyle comportant de 1 à 6 atomes de carbone ; à condition :

(i) que si X représente -CO-, n=2 et B représente un groupe méthyle, A ne représente alors ni un groupe méthyle ni un groupe phényle non substitué ; et
(ii) que si A représente un groupe méthyle, X représente $-COCH_2-$ et n=2, B ne représente alors pas un groupe méthyle ou un groupe phényle.

**2.** Amine cyclique ou sel pharmacologiquement acceptable dérivé de celle-ci, selon la revendication 1, dans laquelle B représente un groupe alkyle comportant de 1 à 6 atomes de carbone.

3. Amine cyclique ou sel pharmacologiquement acceptable dérivé de celle-ci, selon la revendication 1, dans laquelle B représente -Y-Z et Y représente -O-.

4. Amine cyclique ou sel pharmacologiquement acceptable dérivé de celle-ci, selon la revendication 1, dans laquelle A représente un groupe naphtyle ou un groupe naphtyle comportant un substituant choisi parmi groupe alkoxy comportant de 1 à 6 atomes de carbone et un groupe hydroxy, X représente $-CO-CH_2-$ et B représente -Y-Z-, Y représentant -O- ou -NH-, et Z représente un groupe phényle ou un groupe phényle comportant 1 ou 2 substituants qui peuvent être identiques ou différents l'un de l'autre, choisis parmi un atome d'halogène, un groupe alkyle comportant de 1 à 6 atomes de carbone, un groupe alkoxy comportant de 1 à 6 atomes de carbone et un groupe naphtyle.

5. Procédé de préparation d'une amine cyclique ou d'un sel pharmacologiquement acceptable dérivé de celle-ci, selon la revendication 1, comprenant les étapes consistant à faire réagir un composé de type halogénure de formule A-X-Hal dans laquelle Hal représente un atome d'halogène, et A ainsi que X sont respectivement tels que définis dans la revendication 1, avec un composé de formule :

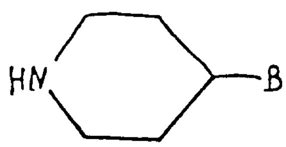

dans laquelle B est tel que défini dans la revendication 1.

6. Composition pharmaceutique comprenant une quantité pharmacologiquement efficace d'une amine cyclique ou d'un sel pharmacologiquement acceptable dérivé de celle-ci, selon la revendication 1, et un véhicule pharmacologiquement acceptable.

7. Utilisation d'une quantité pharmacologiquement efficace d'une amine cyclique ou d'un sel pharmacologiquement acceptable dérivé de celle-ci, représentée par la formule générale :

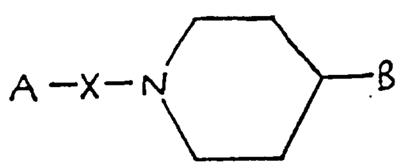

dans laquelle A représente un groupe naphtyle ou un groupe naphtyle substitué avec un groupe alkoxy comportant de 1 à 6 atomes de carbone ou un groupe hydroxy ; un groupe phényle ou un groupe phényle substitué avec un groupe alkyle comportant de 1 à 6 atomes de carbone ou un atome d'halogène ; un groupe quinolyle ; ou un groupe alkyle comportant de 1 à 6 atomes de carbone,
X représente un groupe de formule

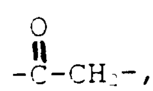

un groupe de formule

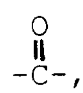

un groupe de formule

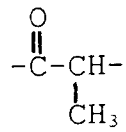

ou un groupe de formule

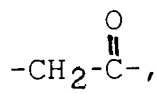

et
B représente un groupe alkyle comportant de 1 à 6 atomes de carbone, ou un groupe de formule -Y-Z dans laquelle Y représente -O- ou -NH- ; et
Z représente un groupe phényle qui peut être substitué avec 1 ou 2 substituants identiques ou différents, choisis parmi un atome d'halogène, un groupe alkyle comportant de 1 à 6 atomes de carbone et un groupe alkoxy comportant de 1 à 6 atomes de carbone ; un groupe naphtyle : un groupe pyridyle ; ou un groupe alkyle comportant de 1 à 6 atomes de carbone ;
pour la préparation d'un médicament destiné à améliorer, à traiter ou à prévenir un symptôme mental associé à une maladie cérébro-vasculaire.